Europäisches Patentamt

European Patent Office

Office européen des brevets

(11) Veröffentlichungsnummer: **0 347 517**

**A1**

# EUROPÄISCHE PATENTANMELDUNG

(21) Anmeldenummer: **89100814.6**

(22) Anmeldetag: **18.01.89**

(51) Int. Cl.⁴: **A61H 23/00 , C08L 33/14 , G10K 11/02**

(30) Priorität: **21.06.88 US 209610**

(43) Veröffentlichungstag der Anmeldung:
**27.12.89 Patentblatt 89/52**

(84) Benannte Vertragsstaaten:
**DE FR GB NL**

(71) Anmelder: **Siemens Aktiengesellschaft**
**Wittelsbacherplatz 2**
**D-8000 München 2(DE)**

(72) Erfinder: **Berg, Joachim**
**31 Hollywood Avenue**
**Leonardo New Jersey 07737(US)**
Erfinder: **Oppelt, Sylvester**
**Greiffenbergstrasse 51**
**D-8600 Bamberg(DE)**

(54) **Wasserhaltiges Gel zur akustischen Ankopplung einer Druckwellenquelle an die Körperoberfläche eines Lebewesens.**

(57) Die Erfindung betrifft ein wasserhaltiges Gel zur akustischen Ankopplung einer Druckwellenquelle (1) an die Körperoberfläche eines Lebewesens. Da das Gel neben Wasser Polyglycerylmethacrylat enthält, sind keine besonderen Zuschlagsstoffe erforderlich, um eine ausreichend hohe Viskosität und eine gute physiologische Verträglichkeit des Gels zu gewährleisten.

EP 0 347 517 A1

# Wasserhaltiges Gel zur akustischen Ankopplung einer Druckwellenquelle an die Körperoberfläche eines Lebewesens

Die Erfindung betrifft ein wasserhaltiges Gel zur akustischen Ankopplung einer Druckwellenquelle an die Körperoberfläche eines Lebewesens.

Derartige Gele werden auf dem Gebiet der medizinischen Diagnose- und Therapiegeräte, die mit Druckwellen, z.B. Ultraschall oder Stoßwellen, arbeiten, dazu verwendet, eine Druckwellenquelle an den Körper des zu untersuchenden bzw. zu behandelnden Patienten akustisch anzukoppeln. Dabei wird derjenige Teil der Körperoberfläche des Lebewesens, mit dem ein Applikationsende der Druckwellenquelle in Kontakt gebracht werden soll, mit dem Gel bestrichen, so daß das Applikationsende der Druckwellenquelle mit der Körperoberfläche des Lebewesens in Kontakt gebracht werden kann, ohne daß zwischen beiden Lufteinschlüsse vorliegen, die zu schädlichen den Diagnose- bzw. Therapieerfolg gefährdenden Reflexionen der Druckwellen führen könnten. Wasserhaltige Gele werden deshalb verwendet, weil diese eine niedrige Dämpfung und eine dem Gewebe von Lebewesen im wesentlichen angepaßte Impedanz aufweisen.

Aus der FR-PS 2 395 006 ist ein wasserhaltiges Gel der eingangs genannten Art bekannt, das im wesentlichen aus einer wässrigen Lösung von Glyzerin besteht. Ein solches Gel besitzt den Vorteil, daß es nur äußerst langsam austrocknet, so daß selbst sehr lange dauernde Diagnose- bzw. Therapievorgänge nicht unterbrochen werden müssen, um den Körper des Lebewesens erneut mit Gel bestreichen zu können. Da jedoch ein ausschließlich aus Wasser und Glyzerin bestehendes Gel eine zu geringe Viskosität aufweist, um an der Körperoberfläche des Lebewesens haften zu können, ist dem bekannten Gel ein polymerer Stoff mit sehr hohem Molekulargewicht, nämlich ein Carboxyvinyl, beigemischt, der eine starke Erhöhung der Viskosität des Gels bewirkt. Da Carboxyvinyle stark sauer reagieren, ist dem bekannten Gel außerdem ein Neutralisator beigemischt, um den pH-Wert des Gels auf physiologisch akzeptable Werte einzustellen. Sowohl durch den im Falle des bekannten Gels erforderlichen polymeren Stoff selbst als auch durch den zu dessen Neutralisierung erforderlichen Neutralisator entstehen zusätzliche Kosten. Außerdem ist die Anwesenheit von stark sauer reagierenden Substanzen in Gelen, die mit der Körperoberfläche von Lebewesen in Berührung kommen, grundsätzlich unerwünscht.

Der Erfindung liegt daher die Aufgabe zugrunde, ein Gel der eingangs genannten Art anzugeben, das die erforderliche Viskosität aufweist, ohne daß dem Gel besondere Stoffe zur Einstellung der Viskosität zugefügt werden müssen, und das keine stark sauer reagierenden Substanzen enthält.

Nach der Erfindung wird diese Aufgabe durch ein Gel gelöst, das neben Wasser Polyglycerylmethacrylat enthält. Ein solches Gel weist die zur Ankopplung einer Druckwellenquelle an die Körperoberfläche eines Lebewesens erforderliche Viskosität auf, ohne daß besondere Stoffe zur Einstellung der Viskosität zugefügt werden müssen und ist demzufolge kostengünstig herstellbar. Die Viskosität kann durch das Mischungsverhältnis von Wasser und Polyglycerylmethacrylat den jeweiligen Anforderungen angepaßt werden. Das erfindungsgemäße Gel reagiert im wesentlichen pH-neutral, ohne daß dem Gel besondere Neutralisatoren zugesetzt werden müssen. Außerdem zeigt das erfindungsgemäße Gel eine äußerst geringe Tendenz zum Austrocknen. Ein für viele Anwendungsfälle geeignetes Mischungsverhältnis von Wasser und Polyglycerylmethacrylat beträgt nach einer Variante der Erfindung etwa 1 : 1.

Gemäß einer Ausführungsform der Erfindung ist das in dem Gel enthaltene Wasser elektrolytfrei. Im Vergleich zu einem unter Verwendung von Ionen enthaltendem Wasser, z.B. Leitungswasser, gebildeten Gel weist ein solches Gel eine erhöhte Stabilität und damit eine nochmals verringerte Tendenz zum Austrocknen auf, da durch die Anwesenheit von Ionen verursachte Störungen der Gelstruktur und chemische Reaktionen ausgeschlossen sind. Außerdem wird durch die Verwendung von elektrolytfreiem Wasser die Gelbildung erleichtert.

Gemäß einer Variante der Erfindung enthält das Gel ein Paraben als Konservierungsmittel. Als Paraben kann in dem Gel wenigstens eine Hydroxybenzoesäure und/oder wenigstens ein Hydroxybenzoesäureester enthalten sein. Eine besonders gute Hautverträglichkeit ergibt sich, wenn als Paraben Hydroxybenzoesäurebutylester in dem Gel enthalten ist.

Das erfindungsgemäße Gel kann mit besonderem Vorteil verwendet werden, wenn als Druckwellenquelle eine fokussierte Stoßwellen abstrahlende Stoßwellenquelle zur Zertrümmerung von Konkrementen im Körper eines Lebewesens vorgesehen ist, deren Applikationsende durch einen mit einem Ausbreitungsmedium für die Stoßwellen gefüllten Sack gebildet ist, der unter Zwischenfügung des wasserhaltigen Gels an die Körperoberfläche des Lebewesens anpreßbar ist. In diesem Falle müssen vergleichsweise große Flächen der Körperoberfläche des Lebewesens mit dem Gel bestrichen werden, so daß es wichtig ist, über ein kostengünstiges Gel verfügen zu können. Außerdem erstreckt sich die Behandlung in der Regel über einen längeren Zeitraum, so daß die Stabilität und die geringe Tendenz zum Austrocknen des erfingunsgemäßen Gels ebenso wie dessen Hautverträglichkeit besonders vorteilhaft zur Wirkung kommt. Die hohe Stabilität und die geringe Tendenz zum Austrocknen des erfindungsgemäßen Gels sind im Falle der Zertrümmerung von Konkrementen außerdem deshalb von besonderer Bedeutung, weil die Stoßwellenquelle relativ zu dem Körper des Lebewesens so ausgerichtet sein muß, daß sich das zu zertrümmernde Konkrement exakt im Fokus der Stoßwellen befindet und somit eine Unterbrechung der Behandlung, um die Körperoberfläche des Lebewesens erneut mit einem Gel zu bestreichen, in der Praxis als äußerst störend empfunden würde, da dann eine erneute Ausrichtung der Stoßwellenquelle erforderlich wäre.

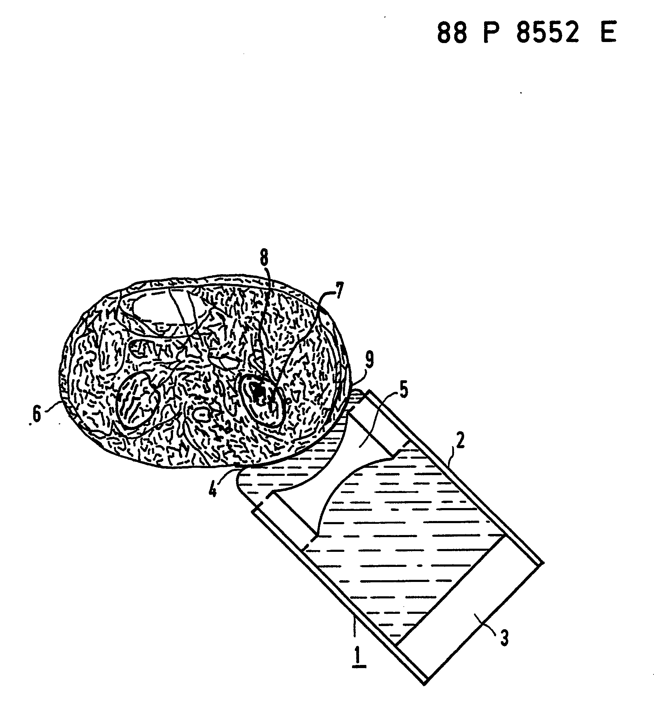

In der beigefügten Zeichnung ist die Verwendung des erfindungsgemäßen Gels beim Zertrümmern von Konkrementen im Körper eines Lebewesens schematisch veranschaulicht.

Die Fig. zeigt eine schematisch angedeutete und insgesamt mit 1 bezeichnete Stoßwellenquelle, wie sie z.B. aus der DE-OS 33 28 051 bekannt ist. Diese besitzt ein mit Wasser als akustisches Ausbreitungsmedium gefülltes rohrförmiges Gehäuse 2, das an seinem einen Ende mit einer Stoßwellenerzeuger 3 versehen und an seinem anderen Ende durch einen flexiblen Sack 4 verschlossen ist. Zwischen dem Stoßwellenerzeuger 3 und dem Sack 4 ist in dem Gehäuse 2 eine akustische Sammellinse 5 angeordnet, die zur Fokussierung der von dem Stoßwellenerzeuger 3 abgegebenen Stoßwellen dient, die im Fokus der Sammellinse 5 zusammenlaufen.

Die Stoßwellenquelle 1 ist mittels des Sackes 4, der das Applikationsende der Stoßwellenquelle 1 bildet, an den Körper 6 eines Lebewesens angepreßt, und zwar derart, daß sich ein in einer Niere 7 des Lebewesens befindlicher Nierenstein 8 im Brennpunkt der Sammellinse 5 befindet. Zwischen dem Sack 4 und dem Körper 6 des Lebewesens befindet sich eine Schicht 9 des erfindungsgemäßen Gels, die zur akustischen Ankopplung der Stoßwellenquelle 1 an den Körper 6 des Lebewesens dient. Die von der Stoßwellenquelle 1 abgegebenen Stoßwellen wirken auf den im Fokus der Sammellinse 5 befindlichen Nierenstein 8 ein und üben mechanische Beanspruchungen auf diesen aus, so daß er in kleine Bruchstücke zerfällt, die auf natürlichem Wege abgehen können.

## Ansprüche

1. Wasserhaltiges Gel zur akustischen Ankopplung einer Druckwellenquelle (1) an die Körperoberfläche eines Lebewesens, **dadurch gekennzeichnet,** daß es neben Wasser Polyglycerylmethacrylat enthält.

2. Wasserhaltiges Gel nach Anspruch 1, **dadurch gekennzeichnet,** daß das Mischungsverhältnis von Wasser und Polyglycerylmethacrylat etwa 1 : 1 beträgt.

3. Wasserhaltiges Gel Anspruch 1 oder 2, **dadurch gekennzeichnet,** daß das in dem Gel enthaltene Wasser elektrolytfrei ist.

4. Wasserhaltiges Gel nach einem der Ansprüche 1 bis 3, **dadurch gekennzeichnet,** daß es wenigstens ein Paraben als Konservierungsmittel enthält.

5. Wasserhaltiges Gel nach 4, **dadurch gekennzeichnet,** daß als Paraben wenigstens eine Hydroxybenzoesäure und/oder wenigstens ein Hydroxybenzoesäureester in dem Gel enthalten ist.

6. Wasserhaltiges Gel nach Anspruch 5, **dadurch gekennzeichnet,** daß als Paraben Hydroxybenzoesäurebutylester in dem Gel enthalten ist.

7. Verwendung eines wasserhaltigen Gels nach einem der Ansprüche 1 bis 6, **dadurch gekennzeichnet,** daß als Druckwellenquelle eine fokussierte Stoßwellen abstrahlende Stoßwellenquelle (1) zur Zertrümmerung von Konkrementen (8) im Körper (6) eines Lebewesens vorgesehen ist, deren Applikationsende durch einen mit einem Ausbreitungsmedium für die Stoßwellen gefüllten Sack (4) gebildet ist, der unter Zwischenfügung einer Schicht (9) des wasserhaltigen Gels an die Körperoberfläche des Lebewesens anpreßbar ist.

88 P 8552 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | FR-A-2 395 006 (DELALANDE S.A.) <br> * Seite 1, Zeile 19; Seite 2, Zeilen 11-13 * <br> --- | 1 | A 61 H 23/00 <br> C 08 L 33/14 <br> G 10 K 11/02 |
| A | US-A-4 646 725 (M. MOASSER) <br> * Spalte 5, Zeilen 60-68 * <br> ----- | 7 | |

**RECHERCHIERTE SACHGEBIETE (Int. Cl.4)**

A 61 H

Der vorliegende Recherchenbericht wurde für alle Patentansprüche erstellt

| Recherchenort | Abschlußdatum der Recherche | Prüfer |
|---|---|---|
| DEN HAAG | 18-05-1989 | GERARD B.E. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

......................................................................
& : Mitglied der gleichen Patentfamilie, übereinstimmendes Dokument

EPO FORM 1503 03.82 (P0403)